# EUROPEAN PATENT APPLICATION

(11) **EP 3 443 934 A1**
(43) Date of publication of application: **20.02.2019**
(21) Application number: 18199410.4
(22) Date of filing: 16.01.2018
(51) Int. Cl.: A61F 2/07, A61F 2/06

(54) **DIAMETER ADJUSTABLE ANEURYSM GRAFT ASSEMBLY**

(30) Priority: 18.01.2017 US 201762447677 P; 09.01.2018 US 201815865919
(62) Divisional of application: 18275006.7
(71) Applicant: Cook Medical Technologies LLC, Bloomington, IN 47404 (US)
(72) Inventor: RASMUSSEN, Erik E., 4200 Slageise (DK); KLAUSEN, Kasper, 4623 Lille Skensved (DK)
(74) Representative: Williams Powell

(57) **Abstract**

A graft material suitable for use in a medical procedure, in one example, material comprises a plurality of circumferential threads (200) and achieves at least two unique physical states. Each thread is constructed such that during a first state, the threads (200) are taut, inexpansible, and have a first diameter. Each thread is also constructed such that during a second state occurring after the first state, the threads (200) are taut, inexpansible, and have a second diameter greater than the first diameter. The first and second diameters are predetermined. In another example, at least one thread is constructed from at least two interwoven fibres, at least one of which is biodegradable. Over time, at least one fibre biodegrades, allowing the remaining fibre(s) to elongate, thereby achieving the second inexpansible state having a greater diameter.

## Description

### BACKGROUND

The disclosed embodiments relate generally to medical devices, and more particularly, to endografts used to treat a diseased vessel or region of vessels.

The functional vessels of human and animal bodies, such as blood vessels and ducts, occasionally weaken or even rupture. For example, the aortic wall can weaken, resulting in an aneurysm. Upon further exposure to hemodynamic forces, such an aneurysm can rupture. One study found that in Western European and Australian men who are between 60 and 75 years of age, aortic aneurysms greater than 29 mm in diameter are found in 6.9% of the population, and those greater than 40 mm are present in 1.8% of the population.

One surgical intervention for weakened, aneurysmal, or ruptured vessels involves the use of an endoluminal prosthesis such as a stent-graft or endograft. Such a prosthesis may provide some or all of the functionality of the original, healthy vessel and/or preserve any remaining vascular integrity by replacing a length of the existing vessel wall that spans the site of vessel failure. A prosthesis of this type can treat, for example, aneurysms of the aortic arch, thoracic aorta, abdominal aortic, iliac, or renal arteries. In cases of aortic pathologies such as dissection or aneurysm, it is often necessary to introduce an endograft to replace or exclude the affected portion of the anatomy. Although open repair to replace a portion of the vessel may be preferable in some cases, many patients are ineligible for open surgery due to secondary issues, and require the placement of an endograft for treatment.

A properly placed prosthesis excludes the diseased and/or aneurysmal portion of the vessel. For weakened or aneurysmal vessels, even a small leak ("endoleak") in or around the prosthesis may lead to the pressurization of or flow (including retrograde flow) in the treated vessel which may aggravate the condition that the prosthesis was intended to treat. Over time, even a properly placed prosthesis may eventually have a reduced hemodynamic seal and incur endoleak in or around the prosthesis, for example, due to changes in anatomy or disease progression.

### SUMMARY

The present invention seeks to provide an improved medical device.

According to an aspect of the present invention, there is provided a medical device as specified in claim 1.

The disclosed embodiments relate to a graft material suitable for use in a medical procedure and it is to be understood that the scope of the disclosure encompasses a graft material suitable for a medical device such as an endograft, as taught herein, which therefore constitutes another claimable aspect of the present invention.

In one example, the graft material may have a plurality of circumferential threads and may achieve at least two unique physical states. Each thread may be constructed such that during a first state, the threads are taut, inexpansible, and have a first diameter. Each thread may also be constructed such that during a second state occurring after the first state, the threads are taut, inexpansible, and have a second diameter greater than the first diameter.

In another example, the first and second diameters may be predetermined.

In another example, at least one thread may be constructed from at least two fibres. The two fibres may be interwoven or intertwined. Each fibre may have a bioabsorption rate. The first fibre may be non-bioabsorbable, and the second fibre may be bioabsorbable. The first fibre may be inexpansible. In the second state having a greater diameter than the first state, the second fibre may be substantially bioabsorbed.

In a further example, at least one thread may further comprise a third fibre, wherein the first and third fibres are counter-woven around the second fibre. The third fibre may be non-bioabsorbable and inexpansible. Alternatively, the third fibre may be inexpansible and have a third bioabsorption rate greater than zero and different from the second bioabsorption rate of the second fibre.

In another example, the graft material may form at least one sealing zone of an endograft. In another example, one of the fibres may comprise texturized yarn.

In another example, the first fibre and second fibre may be interwoven (or intertwined) at a generally uniform interweave spacing. The difference in length between the second circumference and the first circumference may be proportional to the interweave spacing.

The methods and systems disclosed herein are non-limiting and may be applied to other vasculature or anatomy. Other systems, methods, features and advantages of the invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

The teachings herein can be better understood with reference to the following drawings and description of preferred embodiments of the invention, described by way of example only. The components in the figures are not necessarily to scale, emphasis instead being placed upon illustrating the principles of the teachings herein.

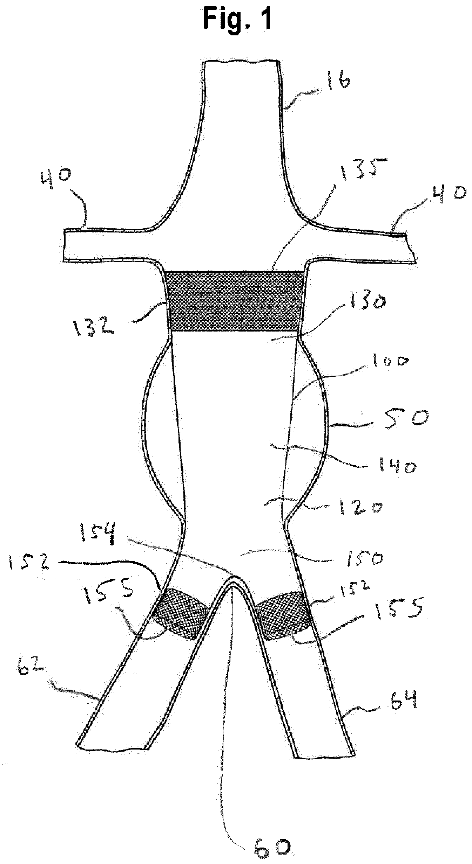

FIG. 1 is an anatomical side view of the descending aorta including the abdominal aorta, renal arteries, iliac bifurcation, and iliac arteries. Within the abdominal aorta is a branched endograft spanning an aneurysm and having at least three sealing zones where the endograft meets the vessel walls.

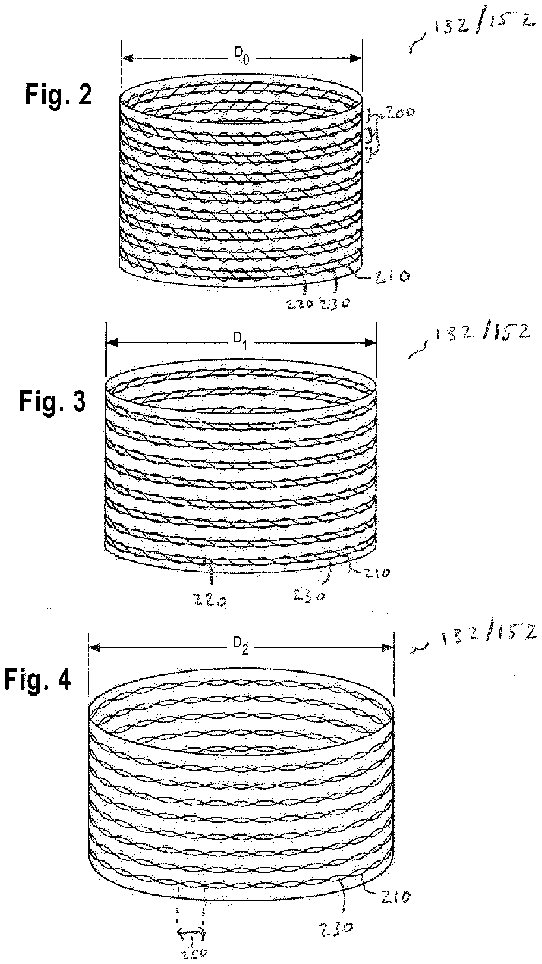

FIGS. 2-4 are side perspective views of an embodiment of a sealing zone of an endograft having an initial diameter D₀, an intermediate diameter D₁, and a final diameter D₂. Each of the plurality of threads within the sealing zone comprises a plurality of fibres woven together and in different states of bioabsorption. For illustrative purposes, only the circumferential threads are shown and the longitudinal threads are hidden.

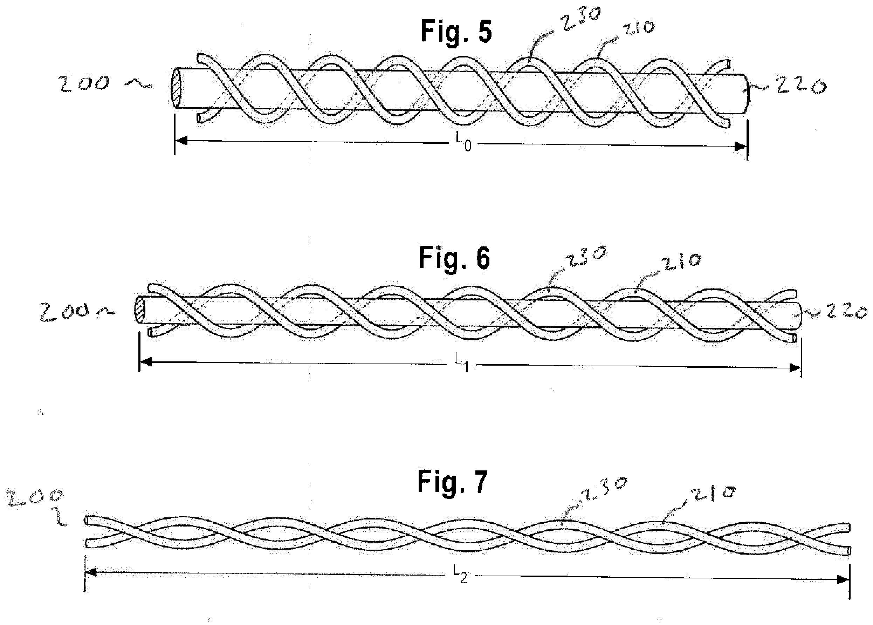

FIGS. 5-7 are side views of an embodiment of a thread having an initial length L₀, an intermediate length L₁, and a final length L₂, corresponding to the initial diameter D₀, intermediate diameter D₁, and final diameter D₂ of FIGS. 2-4. Each thread comprises a plurality of fibres woven together and in different states of bioabsorption.

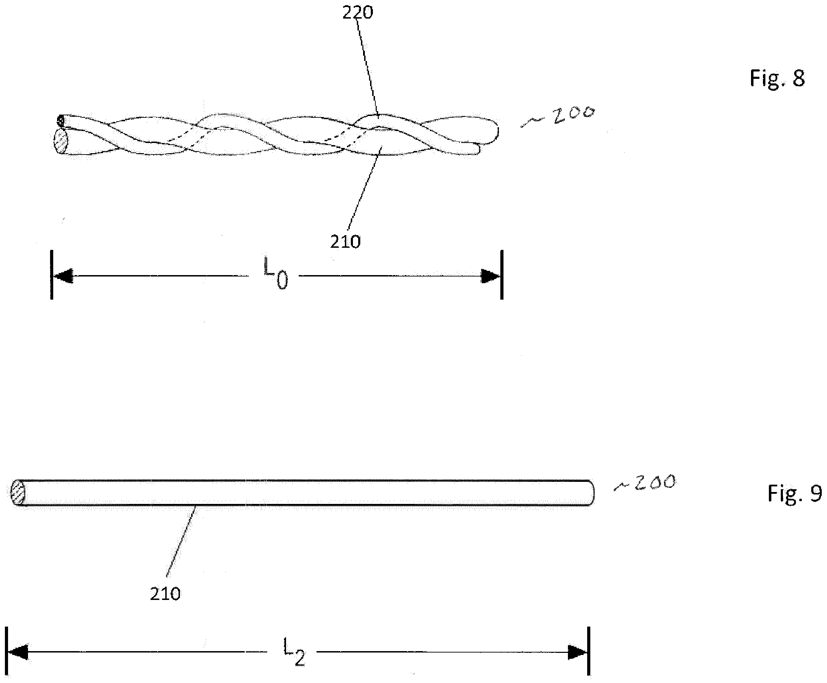

FIGS. 8-9 are side views of an embodiment of a thread having an initial length L₀ and a final length L₂, corresponding to the initial diameter D₀ and final diameter D₂ of a sealing zone of an endograft. Each thread comprises a plurality of fibres woven together and in different states of bioabsorption.

### DETAILED DESCRIPTION OF THE PREFERRED EMBODIMENTS

In the present application, the term "proximal" refers to a direction that is generally upstream to the direction of blood flow during a medical procedure, while the term "distal" refers to a direction that is generally downstream to the direction of blood flow during a medical procedure.

As used herein, the terms "bioabsorption," "biodegradable," and "bioabsorbable" mean a capacity to be taken in and made part of an existent whole, to be assimilated, or to be dissolved by an organism. Per the purposes of this disclosure, "bioabsorption," "biodegradable," "bioresorbable," "bioerodable," and "bioabsorbable" have the same meaning. It is to be understood that wherever the term "bioabsorbable" is used, including the claims, the terms "bioabsorption," "bioresorbable," "bioerodable," and "biodegradable" can be substituted.

The embodiments described below are in connection with systems and methods for the introduction and deployment of an implantable medical device in a vessel, such as endovascular prostheses, but could also be used for deploying a range of implantable medical devices including, but not limited to, stents, occlusion devices and the like.

Referring to FIG. 1, the aorta 10 is the largest artery in the human body and carries blood away from the heart, including through the descending aorta 16. Over time, the walls of the aorta 10, descending aorta 16, and other vessels may lose elasticity or otherwise weaken. Due to hemodynamic pressure, the vessel walls may expand in diameter, resulting in an aneurysm 50. While an aneurysm by itself is not an acute problem, it can increase the risk of a possibly fatal vessel rupture if the aneurysm expands and/or bursts. A common treatment for the aneurysm is to relieve the pressure on the aneurysm by redirecting blood flow through a stent graft or endograft 100. As illustrated, the endograft 100 has two equal length "legs." However, the "legs" may not necessarily be the same length. The concepts illustrated herein may be applied to any endograft, including but not limited to, the family of Zenith Flex® AAA Endovascular Grafts (Cook Medical Inc.), the family of Zenith® TX2® TAA Endovascular Grafts (Cook Medical Inc.), and endografts for treating abdominal aortic aneurisms ("AAA"), thoracic abdominal aortic aneurisms ("TAAA"), and thoracic aortic aneurisms ("TAA").

An aneurysm 50 may form at almost any level of the aorta or other vessel, for example, in the descending aorta 16 distal to the renal arteries 40 and proximal to the iliac bifurcation 60. As shown, an endograft 100 may be implanted in the aorta 10 (here, descending aorta 16), such that blood flows through the endograft 100, avoiding the aneurysm 50. Use of an endograft 100 reduces pressure on the aneurysm 50 and can cause the aneurysm 50 to shrink in size. The endograft 100 may incorporate self-expanding stents. The shape, size, and position of the endograft 100 may also be modified through use of a balloon catheter.

The endograft 100 may comprise an expandable support structure 110 (not shown) and a graft material 120, including a tubular main body 140 having a proximal end 130 with a proximal opening 135, a distal end 150 with a distal bifurcation 154 and distal openings 155, and a lumen 160 extending therebetween. The proximal opening 135 and distal openings 155 may both provide fluid access to the lumen 160 of the main body 140. The main body 140 may be generally tubular in shape, and have either a uniform or varying diameter along its length.

The proximal end 130 may have a proximal sealing zone 132 where the graft material 120 is in contact with a vessel wall, here, the descending aorta 16 distal to the renal arteries 40 and proximal to the iliac bifurcation 60. The distal end 150 may have distal sealing zones 152 where the graft material 120 is in contact with the vessel walls, here, the right iliac artery 62 (shown on left) and left iliac artery 64 (shown on right) distal to the iliac bifurcation. The proximal and distal sealing zones 132 and 152, respectively, may form a hemodynamic seal with their respective vessel walls. The hemodynamic seal may prevent endoleak, retrograde blood flow, and blood flowing past (exerting pressure on) the aneurysm 50. This may also help maintain the hemodynamic seal over a longer period of time and over a range of conditions.

In another embodiment (not shown), the proximal end 130 may extend proximally such that the proximal sealing zone 132 is proximal to the renal arteries 40. To maintain blood flow to the renal arteries 40, the main body 140 may have one or more openings called fenestrations. Covered branch stents (or branch endografts) may extend through one or more fenestrations to cannulate the renal arteries 40. The branch stent may be deployed with the distal end within the renal arteries 40, the branch stent body passing through a fenestration in the endograft 100, and some proximal portion of the branch stent protruding into the lumen 160 of the endograft 100.

Referring to FIGS. 2-4, the graft material 120 at the proximal and distal sealing zones 132 and 152, respectively, may differ from the graft material in the remainder of the endograft 100. The graft material 120 at the sealing zones 132 and 152 may be woven from a plurality of circumferential threads 200. Each of the circumferential threads may comprise interwoven fibres (or intertwined fibres) that may have different properties, for example, different degrees of elasticity, biodegradability, etc. For illustrative purposes, FIGS. 2-4 only shows the circumferential threads, and the longitudinal threads are hidden. The longitudinal threads are interwoven (or intertwined) with the circumferential threads to form the graft material 120. The interweaving pattern found at the sealing zones 132 and 152 may be the same pattern as found throughout the remainder of the graft. The longitudinal threads may be formed using any appropriate biocompatible material, for example, polyester yarn.

Referring to FIGS. 5-7, each figure shows an individual thread from corresponding FIGS. 2-4. FIG. 5 shows a thread from FIG. 2, FIG. 6 shows a thread from FIG. 3, and FIG. 7 shows a thread from FIG. 4.

The circumferential threads 200 may comprise at least first and second fibres 210 and 220, respectively, having first and second bioabsorption rates 212 and 222 and first and second spring constants 214 and 224, respectively. As shown in FIGS. 2-3 (and FIGS. 5-6), the circumferential threads 200 may further comprise a third fibre 230 having a third bioabsorption rate 232 and third spring constant 234. The second fibre 220 may form a coaxial core, around which the first fibre 210 and third fibre 230 are counter-woven. Other patterns may also be utilized, for example, criss-crossing fibres, various braid patterns, etc.

For example, the first and third fibres 210 and 230, respectively, may have first and third bioabsorption rates 212 and 232 of about zero, such that both are substantially non-bioabsorbable (non-biodegradable). The first and third fibres 210 and 230, respectively, may have first and third spring constants 214 and 234 of about zero, such that both are substantially inelastic. The second fibre 220 may have a second bioabsorption rate 222 greater than zero, such that it may biodegrade over time after implantation. The second fibre 220 may either have a second spring constant 224 of about zero (substantially inelastic) or greater than zero (elastic).

After endograft 100 is deployed (post-implantation), any fibres exposed to the body and having an absorption rate greater than zero may biodegrade as molecules of the fibre are absorbed into the body over time. Continuing with the example from above, the second fibre 220 having a second bioabsorption rate 222 greater than zero may biodegrade over time while first and third fibres 210 and 230, respectively, having bioabsorption rates (212 and 232) of about zero do not biodegrade. As a result, the length of each thread will elongate from an initial length L₀ and diameter D₀ (FIGS. 2, 5), to an intermediate length L₁ and diameter D₁ as the second fibre is partially biodegraded (FIGS. 3, 6), to a final length L₂ and diameter D₂ as the second fibre is completely biodegraded (FIGS. 4, 7). In this example, since the first and third fibres 210 and 230, respectively, have first and third spring constants 214 and 234 of about zero (substantially inelastic or inexpansible), L₂ (and corresponding diameter D₂) is the length of the first fibre 210 and third fibre 230 counter-woven around one another.

In one example, D₀ may be about 36mm, D₂ may be 40-42mm, and D₁ may be any intervening diameter. Other sizes can be easily made to accommodate a patient's specific anatomy and anticipated changes in diameter.

The lengths L₀ and L₂ can be predetermined by precisely winding the first fibre 210 and third fibre 230 around the second fibre 220. The first fibre 210 and third fibre 230 may be counter-woven and have a generally uniform interweave spacing 250. The greater the frequency that first fibre 210 and third fibre 220 are wound around second fibre 220, the greater the difference between lengths L₀ and L₂ (and diameters D₀ and D₂). This is because each additional winding provides additional fibre length that will become available after the second fibre 220 biodegrades. The interweave spacing may be directly proportional to the change in length from L₀ to L₂. Additionally, since the first fibre 210 and third fibre 230 are substantially inelastic (and the second fibre 220 may also be substantially inelastic or inexpansible), the lengths L₀ and L₂ may be substantially inexpansible.

As shown in FIGS. 5-7, each of the circumferential threads 200 may elongate from an initial length L₀, to an intermediate length L₁, to a final length L₂. Thus, the diameter of the endograft body at the proximal and distal sealing zones 132 and 152, respectively, will increase from an initial diameter D₀, to an intermediate diameter D₁, to a final diameter D₂, as shown in FIGS. 2-4. As discussed herein, diameters D₀, D₁, and D₂ may be general notations for the changing diameter at a given sealing zone and specific to the surrounding anatomy. For example, diameter D₀ at the proximal sealing zone 132 in the descending aorta 16 may be greater than D₀ at the distal sealing zone 152 in the iliac arteries 62 and 64, since the descending aorta 16 is wider than the iliac arteries 62 and 64. Similarly, lengths L₀, L₁, and L₂ may be general notations for the changing lengths for a given portion of thread.

In the same manner that the difference between L₀ and L₂ can be predetermined by precisely winding the first and third fibres 210 and 230 around the second fibre 220, D₀ and D₂ can be similarly predetermined since circumferential thread length is directly proportional to diameter (circumference = π * diameter). Thus, the initial diameter D₀ and final diameter D₂ can be predetermined by controlling frequency of the fibre windings.

Other factors that may affect the difference of D₀ and D₂ include the diameter of second fibre 220, and the spacing of the windings (windings per unit length) of 230 and 210 around the second fibre 220. The difference between D₀ and D₂ may increase for larger diameters of second fibre 220 and for greater numbers of windings per unit length of first fibre 210 and third fibre 230. Another factor that may affect the difference between D₀ and D₂ is the exact woven textile of the graft material 120. A more rigid graft material 120 may reduce the flexibility of the graft material at the sealing zones.

In general, the diameter of the first fibre 210 and third fibre 230 may be between 33% and 200% the diameter of the second fibre 220. However, these ranges are not absolute, and may be between 1% and 1000% of the second fibre 220.

The rate of change in diameter can be predetermined by controlling the bioabsorption rate 222 of the second fibre 220. In essence, the higher the bioabsorption rate 222, the faster the diameter will increase from D₀ to D₂. Another factor that may affect the rate of change in diameter is the spacing of the windings, which may also affect the bioabsorption rate 222 of the second fibre 220. For example, if the first fibre 210 and third fibre 230 are counter-woven tightly (increased windings per unit length) around the second fibre 220, the surface area of the second fibre 220 actually exposed to blood (or other fluids) may be reduced, thereby reducing the actual bioabsorption rate 222. This may be further affected by the material and construction of the fibres themselves (e.g., hydrophobic vs. hydrophilic). Thus, the optimal spacing of the first and third fibres 210 and 230 around the co-axial core second fibre 220 may require empirical testing for the given material(s) used to construct the threads.

The selection of material for second fibre 220 may produce graft materials 120 with varying mechanical properties at the sealing zones. For example, a polycaprolactone or a copolymer of polycaprolactone may provide higher elasticity of second fibre 220 (or other components of the graft material 120), while other materials such as Polylactide, Polyglycolide, or a co-polymer thereof, may provide lower elasticity of second fibre 220 (or other components of the graft material 120).

The expandable support structure 110 provides a constant expansion force throughout the endograft body 140, including the proximal sealing zone 132 and distal sealing zones 152. Thus, as the fibres biodegrade, the threads gradually elongate, and the diameter at the sealing zones gradually increase because the graft material is subject to radially outward tension via the expandable support structure 110.

The support structure 110 of the endograft 100 may have any suitable stent pattern known in the art. The support structure 110 may be self-expanding or may expand under external pressures, for example from an inflatable balloon at the tip of a balloon catheter. One example of a stent pattern is the Z-stent or Gianturco stent design. Each Z-stent may include a series of substantially straight segments or struts interconnected by a series of bent segments or bends. The bent segments may include acute bends or apices. The Z-stents are arranged in a zigzag configuration in which the straight segments are set at angles relative to one another and are connected by the bent segments. Other stents may include, for example, annular or helical stents. The stents mentioned herein may be made from standard medical grade stainless steel. Other stents may be made from nitinol or other shape-memory materials.

The graft material 120 may be connected to the one or more support structures 110 (not shown) by known methods, for example biocompatible stitching. The graft material 120 for the main endograft body 140 (excluding the proximal and distal sealing zones 132 and 152, respectively) may be fabricated from any at least substantially biocompatible material including such materials as polyester fabrics, polytetrafluoroethylene (PTFE), expanded PTFE, and other synthetic materials known to those of skill in the art. In some embodiments in accordance with the technology, the graft material 120 may also include drug-eluting coatings or implants. These materials may also be used in fabricating the non-biodegradable fibres used at the sealing zones (e.g., first fibre 210, third fibre 230, etc.).

The biodegradable fibres used in the threads comprising the plurality of circumferential threads 200 used at the proximal and distal sealing zones 132 and 152, respectively, may be fabricated from any at least substantially biocompatible and biodegradable material including such materials as polyhydroxyalkanoate, poly(alphahydroxy acid) such as polylactide (PLA) [including poly-L-lactide (PLLA), poly-D-lactide (PDLA)], polyglycolide (PGA), polydioxanone, polycaprolactone, polygluconate, polylactic acid-polyethylene oxide copolymers, poly(hydroxybutyrate), polyanhydride, polyphosphoester, poly(amino acids), and/or related copolymers thereof, each of which have a characteristic degradation rate in the body. For example, PGA and polydioxanone are relatively fast-bioabsorbing materials (weeks to months) and PLA and polycaprolactone are relatively slow- bioabsorbing material (months to years). Other synthetic materials known to those of skill in the art may also be utilized. In some embodiments in accordance with the technology, the biodegradable fibres may also include drug-eluting coatings or implants.

In some examples, relatively slow bioabsorbing materials may be preferable. For example, in patients where the predicted rate of expansion of the vessel is on the order of months to years, materials and fabrications that biodegrade at a similar rate may be utilized so as to maintain the hemodynamic seal over the same time period. Additionally, this may allow tissue ingrowth into the newly created space where the bioabsorbable materials have biodegraded, thereby maintaining the fluid-tight seal at the sealing zone and preventing the graft material from leaking or sweating.

In one example, the bioabsorption may occur over the course of 2-6 months. In another example, the bioabsorption may occur over the course of 6-12 months. In another example, the bioabsorption may occur over the course of 1-5 years.

As shown in FIGS. 8-9, in another example, the plurality of circumferential threads 200 at the proximal and distal sealing zones 132 and 152, respectively, may each comprise two fibres, a first fibre 210 and a second fibre 220. As shown, the first fibre 210 and the second fibre 220 may be interwoven or intertwined. The first and second fibres 210 and 220 may have first and second bioabsorption rates 212 and 222, and first and second spring constants 214 and 224, respectively. For example, the first fibre 210 may have a bioabsorption rate 212 of about zero, such that it is substantially non-bioabsorbable (non-biodegradable). The first fibre 210 may have spring constant 214 of about zero such that it is substantially inelastic. The second fibre 220 core may have a second bioabsorption rate 222 greater than zero, such that it may biodegrade over time after implantation. The second fibre 220 may either have a second spring constant 224 of about zero (substantially inelastic or inexpansible) or greater than zero (elastic).

As shown in FIGS. 8-9 and similar to the examples discussed above, after endograft 100 is deployed (post-implantation), the second fibre 220 having a second bioabsorption rate 222 greater than zero may biodegrade as molecules of the fibre are absorbed into the body over time. As described above, the second fibre 220 having a second bioabsorption rate 222 greater than zero may biodegrade over time while the first fibre 210 having a first bioabsorption rate 212 of about zero may not biodegrade. As a result, the length of each thread will elongate from an initial length L₀ (FIG. 8), to an intermediate length L₁ as the second fibre is partially biodegraded (not shown), to a final length L₂ as the second fibre 220 is completely biodegraded and the first fibre 210 straightens and elongates (FIG. 9). In this example, since the first fibre 210 has a spring constant 214 of about zero (substantially inelastic or inexpansible), L₂ may be approximately equal to the length the first fibre 210.

As discussed above with respect to FIGS. 2-7, the change in thread length from an initial length L₀ (FIG. 8), to an intermediate length L₁ (not shown), to a final length L₂ (FIG. 9) corresponds to changes in the diameter of the endograft body at the proximal and distal sealing zones 132 and 152, respectively, which change from an initial diameter D₀, to an intermediate diameter D₁, to a final diameter D₂.

In other examples not shown here, additional fibres may be incorporated into the threads comprising the plurality of circumferential threads 200. For example, a fourth or fifth fibre may be added, each having any of the properties described above with respect to bioabsorption and elasticity (expansibility).

In another example, at least one of the fibres (e.g., first fibre 210 and/or third fibre 230) may be elastic, and as a result, the graft material 120 forming at least one of the proximal or distal sealing zones 132 and 152, respectively, may be elastic and/or expansible so as to accommodate further radial expansion.

In another example, at least two of the fibres (e.g., second fibre 220 and third fibre 230) may have bioabsorption rates greater than zero and that are different from one another. Thus, the threads may have a first predetermined length L₀ (with corresponding first predetermined diameter D₀ at the respective sealing zone) upon initial deployment/implantation. As the fibre with faster bioabsorption rate biodegrades, the threads may have an intermediate length L₁ (with corresponding diameter D₁), and after it completely biodegrades, a second predetermined length L₂ (with corresponding second predetermined diameter D₂ at the respective sealing zone). As the fibre with the slower bioabsorption rate biodegrades, the threads may have an second intermediate length L₃, and after it completely biodegrades, a third predetermined length L₄ (with corresponding third predetermined diameter D₄ at the respective sealing zone). If any of the fibres are inelastic, then the predetermined lengths and diameters may also be inexpansible, whereas elastic fibres may result in expansible lengths and diameters. Additional predetermined lengths (e.g., fourth predetermined length and diameter) can be accommodated via additional fibres having appropriate windings and bioabsorption rates.

In another example, one or more of the fibres (e.g., first fibre 210 and/or second fibre 220) may be formed from a texturized yarn, for example, having a wave-like pattern that may straighten and elongate given appropriate pressure and space to expand. Thus, the threads may have a first predetermined length (and corresponding first predetermined diameter at the respective sealing zone). After the texturized yarn elongates to full length, the threads may have a second predetermined length (and corresponding second predetermined diameter at the respective sealing zone).

The controlled expansion of select portions of an endograft 100 may have several advantages and uses in different patient populations.

First, it may be useful in patients where the aneurysm disease state is predicted to progress towards one or more of the proximal or distal sealing zones 132 and 152, reducing the integrity of the hemodynamic seal. Using such an embodiment may reduce the frequency of follow up procedures which might otherwise be required to repair or replace the endograft 100 when the hemodynamic seal fails (resulting in possible endoleak, retrograde blood flow, etc.). The controlled expansion may result in a hemodynamic seal being maintained even where the aneurysm progresses and the diameter of the vessel at the sealing zone increases. This maintains the fluid-tight seal even as the vessel expands over time, which may not have been possible if the graft material 120 had simply been "oversized." Oversizing the graft material 120 would likely have resulted in folds in the graft material 120, reduced patency with the vessel wall, and a corresponding reduced integrity of the hemodynamic seal.

Second, it may be useful in patients whose bodies that are growing, for example, children or adolescents, where the diameter of certain vessels may grow at predictable rates. Using such an embodiment may reduce the frequency of follow up procedures which might otherwise be required as the patient's cardiac anatomy matures and grows. In such an embodiment, the plurality of circumferential threads 200 comprising first and second fibres 210 and 220, respectively, may be used throughout the construction of the endograft 100, thereby allowing the entire endograft 100 to expand over time, not just the sealing zones 132 and 152 as described in certain examples above.

Third, the controlled bioabsorption may facilitate tissue ingrowth into the endograft 100. Over time, endothelial-like cells may adhere to the endograft 100, resulting in tissue ingrowth. The rate of tissue ingrowth may depend on multiple variables, including graft material, pore size, and thread weave pattern. The bioabsorption rate of the fibres that may biodegrade (e.g., bioabsorption rate 222 of second fibre 220) may be tailored to approximate the tissue in-growth rate. By doing so, the graft material 120 at the sealing zones 132 and 152 may retain sufficient thickness, porosity, and structural integrity.

In use, endograft 100 may be deployed intravascularly, for example in the descending aorta 16 distal to the renal arteries 40. The delivery system containing the graft may be tracked from a distal approach (e.g., transfemoral) and guided to the descending aorta 16 distal to the renal arteries 40. The main body 140 of the endograft 100 may be deployed by retraction of an outer sheath (not shown). The endograft 100 may employ diameter reduction ties (not shown) to prevent full deployment at this stage. After cannulation of any branch vessels (if applicable), if diameter reduction ties are in place, they may be removed to fully deploy the endograft 100. After deployment, any wires and catheters may be removed from the system.

Upon the initial deployment, the graft material may be fully expanded to a first configuration. The plurality of circumferential threads 200 at the proximal and distal sealing zones 132 and 152, respectively, may have an initial predetermined circumference corresponding to the initial diameters D₀ and thread lengths L₀. Over time, one or more biodegradable fibres within each thread (e.g., second fibre 220) may partially biodegrade, resulting in a thinner biodegradable fibre(s) that is still intact. As a result, other fibres interwoven with the at least one biodegradable fibre may elongate and straighten, resulting a longer thread length L₁ and corresponding diameter D₁ at the respective sealing zone(s). After more time, the one or more biodegradable fibres within each thread may completely biodegrade, whereby the remaining fibres may further straighten and elongate. The thread length may increase to a second predetermined length L₂ and the diameter may increase to predetermined diameter D₂ at the respective sealing zone(s). If additional biodegradable fibres are utilized in the fabrication of the threads, additional time may result in the further elongation of threads to L₃ and L₄ and increase in diameter at the respective sealing zones to D₃ and D₄.

Each predetermined thread length (and corresponding predetermined diameter and/or circumference) may be referred to as a "state". For example, the first state may correspond to predetermined thread length L₀ and predetermined diameter D₀, and the second state may correspond to predetermined thread length L₂ and predetermined diameter D₂. As discussed herein, the length of each circumferential thread may increase (e.g., L₀ to L₁ to L₂) and the corresponding diameter at the respective sealing zone may also increase (e.g., D₀ to D₁ to D₂) with a corresponding increase in circumference (e.g., C₀ to C₁ to C₂, not shown).

It should be understood that the circumference (and by association the length of circumferential threads) is generally equivalent to the diameter multiplied by pi. However, anatomical variations and other factors such as materials and fabrication technique may result in some variation. For example, variation in the vessel walls and/or progression of an uneven aneurysm may result in the cross-section of the vessel at the respective sealing zone not being a perfect circle. Thus, herein the diameter may be referenced and discussed for illustrative purposes, it should be understood that any predetermined diameter (e.g., D₀ to D₁ to D₂) implies a predetermined circumference (e.g., C₀ to C₁ to C₂, not shown).

The embodiments described herein provide non-limiting examples of endografts that are suitable for treating an array of medical conditions, and may be especially suited for treating an aortic aneurysm within the descending aorta 16. Various additional modular components may be provided for the endograft 100, for example, additional fenestrations, branching, and branch stents may be utilized.

While references to treatment of an aneurysm at or near the descending aorta 16 may be explained as one example, it will be appreciated that endograft 100 can be positioned at other bodily locations to treat aneurysms or other conditions, using the system and methods described herein.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

The teachings herein also extend to the following aspects of the disclosed invention, covered in parent European patent application number EP18275006.7, of which this is a divisional application.

A said aspect of the invention provides a medical device for treatment of a body vessel, the medical device comprising:
a self-expanding stent support structure;
a tube of graft material connected to the stent support structure and having a proximal end, a distal end, and a main body between the proximal and distal ends,
wherein at least one of the proximal and distal ends comprises a sealing zone having a plurality of circumferential threads interwoven with a plurality of longitudinal threads,
wherein the plurality of circumferential threads are at least partially bioabsorbable,
wherein the plurality of circumferential threads are constructed and dimensioned such that during a first state, the plurality of circumferential threads are taut, inexpansible, and have a first diameter, and during a second state occurring after the first state, the plurality of circumferential threads are at least partially biodegraded, taut, inexpansible, and have a second diameter greater than the first diameter.

Preferably, the at least one circumferential thread comprises a first fibre and a second fibre.

The first fibre and second fibre may be interwoven.

Advantageously, the first fibre and second fibre have different bioabsorption rates. For example, the first fibre may be non-bioabsorbable, and the second fibre may be bioabsorbable.

In preferred embodiments, the first fibre is inexpansible.

Advantageously, at the second state, the second fibre is substantially bioabsorbed.

The medical device may comprise a third fibre, wherein the first and third fibres are counter-woven around the second fibre. Preferably, the third fibre is non-bioabsorbable and inexpansible. The third fibre may have a bioabsorption rate different from the second fibre and is inexpansible.

Preferably, at least one of the first fibre and second fibre comprises texturized yarn.

It is preferred that the graft material forms at least one sealing zone of an endograft.

The disclosures in US provisional patent application No. 62/447,677 filed on January 18, 2017, US non-provisional patent application No. 15/865,919 filed on January 9, 2018, from which this application claims priority, in European patent application number EP18275006.7, of which this is a divisional application, and in the abstract accompanying this application are incorporated by herein reference.

## Claims

1. An endograft for placement in a vessel of a patient, comprising:
an expandable support structure;
a multi-state graft material attached to the expandable support structure to form at least a portion of an endograft body,
wherein the multi-state graft material is constructed such that during a first state, the multiple-state graft material has a first predetermined inelastic circumference, and during a second state that occurs after the first state, the multi-state graft material has a second predetermined inelastic circumference greater than the first circumference.

2. An endograft according to claim 1, wherein the first state occurs after an initial expansion of the expandable support structure during a delivery step.

3. An endograft according to claim 1 or 2, wherein the multi-state graft material has at least one circumferential thread comprising a first fibre having a first bioabsorption rate of about zero, and a second fibre having a second bioabsorption rate greater than zero, and where the first fibre and second fibre are interwoven at a generally uniform interweave spacing.

4. An endograft according to claim 3, wherein the first fibre is substantially inelastic.

5. An endograft according to claim 3 or 4, further comprising a third fibre that is substantially inelastic, wherein the first fiber and third fiber are counterwoven around the second fibre.

6. An endograft according to claim 3, 4 or 5, wherein the difference in length between the second circumference and the first circumference is proportional to the interweave spacing.
